# EUROPEAN PATENT SPECIFICATION

(11) **EP 4 633 728 B1**
(45) Date of publication and mention of the grant of the patent: **14.10.2026**
(21) Application number: 23848617.9
(22) Date of filing: 21.12.2023
(51) Int. Cl.: A61N 1/05, A61B 5/00, A61N 1/36

(54) **NEUROMODULATION SYSTEM WITH ADAPTABLE SENSING AND CLOSED LOOP CONTROL**
NEUROMODULATIONSSYSTEM MIT ANPASSUNGSFÄHIGER SENSORIK UND GESCHLOSSENEM REGELKREIS
SYSTÈME DE NEUROMODULATION AVEC DÉTECTION ADAPTABLE ET CONTRÔLE EN BOUCLE FERMÉE

(30) Priority: 09.01.2023 US 202363479070 P
(43) Date of publication of application: 22.10.2025
(73) Proprietor: Boston Scientific Neuromodulation Corporation, Valencia, CA 91355 (US)
(72) Inventor: HADDOCK, Andrew, Los Angeles, CA 90027 (US); JAYAKUMAR, Adarsh, Valencia, CA 91355 (US); WITWER, Max, Santa Clarita, CA 91355 (US); GREENE, Keron, Valencia, CA 91381 (US)
(74) Representative: Vossius & Partner Patentanwälte Rechtsanwälte mbB
(86) International application number: PCT/US2023/085485
(87) International publication number: WO 2024/151412

(56) References cited:
- US-A1- 2010 211 135
- US-A1- 2022 266 027
- US-B2- 10 786 676
- US-B2- 7 869 881
- US-B2- 9 974 959
- HOSAIN MD KAMAL ET AL: "Closed loop deep brain stimulation: an evolving technology", AUSTRALASIAN PHYSICAL AND ENGINEERING SCIENCES IN MEDICINE, MEDICINE, MELBOURNE, AU, vol. 37, no. 4, 7 September 2014 (2014-09-07), pages 619 - 634, XP035428098, ISSN: 0158-9938, [retrieved on 20140907], DOI: 10.1007/S13246-014-0297-2
- ROUSE A G ET AL: "A chronic generalized bi-directional brainmachine interface;A chronic generalized bi-directional brain--machine interface", JOURNAL OF NEURAL ENGINEERING, INSTITUTE OF PHYSICS PUBLISHING, BRISTOL, GB, vol. 8, no. 3, 5 May 2011 (2011-05-05), pages 36018, XP020205978, ISSN: 1741-2552, DOI: 10.1088/1741-2560/8/3/036018

## Description

### FIELD OF THE INVENTION

This application relates to Implantable Medical Devices (IMDs), and more specifically sensing signals and closed loop feedback in an implantable stimulator device.

### INTRODUCTION

Implantable neurostimulator devices are implantable medical devices (IMDs) that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516227. Other similar neuro-stimulation systems are disclosed, for instance, in US 10,786,676 B2, US 2022/266027 A1, US 9,974,959 B2, US 7,869,881 B2, or US 2010/211135 A1. However, the present invention may find applicability with any implantable neurostimulator device system.

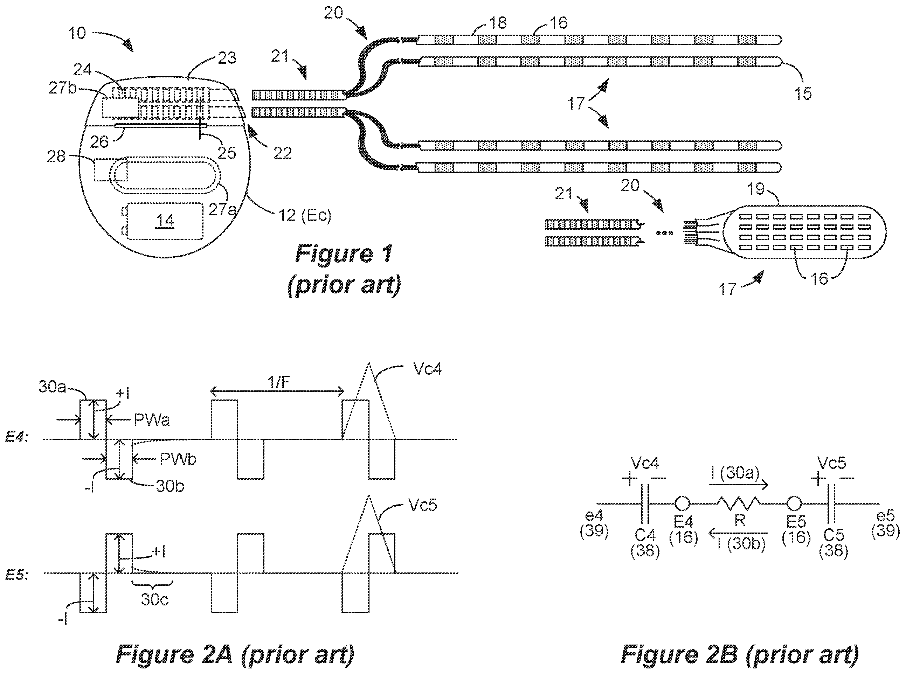

An SCS system typically includes an Implantable Pulse Generator (IPG) 10 shown in Figure 1. The IPG 10 includes a biocompatible device case 12 that holds the circuitry and a battery 14 for providing power for the IPG to function. The IPG 10 is coupled to tissue-stimulating electrodes 16 via one or more electrode leads that form an electrode array 17. For example, one or more percutaneous leads 15 can be used having ring-shaped or split-ring electrodes 16 carried on a flexible body 18. In another example, a paddle lead 19 provides electrodes 16 positioned on one of its generally flat surfaces. Lead wires 20 within the leads are coupled to the electrodes 16 and to proximal contacts 21 insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12.

In the illustrated IPG 10, there are thirty-two electrodes (E1-E32), split between four percutaneous leads 15, or contained on a single paddle lead 19, and thus the header 23 may include a 2x2 array of eight-electrode lead connectors 22. However, the type and number of leads, and the number of electrodes, in an IPG is application-specific and therefore can vary. The conductive case 12 can also comprise an electrode (Ec). In a SCS application, the electrode lead(s) are typically implanted in the spinal column proximate to the dura in a patient's spinal cord, preferably spanning left and right of the patient's spinal column. The proximal contacts 21 are tunneled through the patient's tissue to a distant location such as the buttocks where the IPG case 12 is implanted, at which point they are coupled to the lead connectors 22. In other IPG examples designed for implantation directly at a site requiring stimulation, the IPG can be lead-less, having electrodes 16 instead appearing on the body of the IPG 10 for contacting the patient's tissue. The IPG lead(s) can be integrated with and permanently connected to the IPG 10 in other solutions. The goal of SCS therapy is to provide electrical stimulation from the electrodes 16 to alleviate a patient's symptoms, such as chronic back pain.

IPG 10 can include an antenna 27a allowing it to communicate bi-directionally with a number of external devices used to program or monitor the IPG, such as a hand-held patient controller or a clinician's programmer, as described for example in U.S. Patent Application Publication 2019/0175915. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Zigbee, MICS, and the like.

Stimulation in IPG 10 is typically provided by pulses each of which may include a number of phases such as 30a and 30b, as shown in the example of Figure 2A. Stimulation parameters typically include amplitude (current I, although a voltage amplitude V can also be used); frequency (F): pulse width (PW) of the pulses or of its individual phases; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E4 has been selected as an anode (during its first phase 30a), and thus provides pulses which source a positive current of amplitude +I to the tissue. Electrode E5 has been selected as a cathode (again during first phase 30a), and thus provides pulses which sink a corresponding negative current of amplitude -I from the tissue. This is an example of bipolar stimulation, in which only two lead-based electrodes are used to provide stimulation to the tissue (one anode, one cathode). However, more than one electrode may be selected to act as an anode at a given time, and more than one electrode may be selected to act as a cathode at a given time. The case electrode Ec (12) can also be selected as an electrode, or current return, in what is known as monopolar situation.

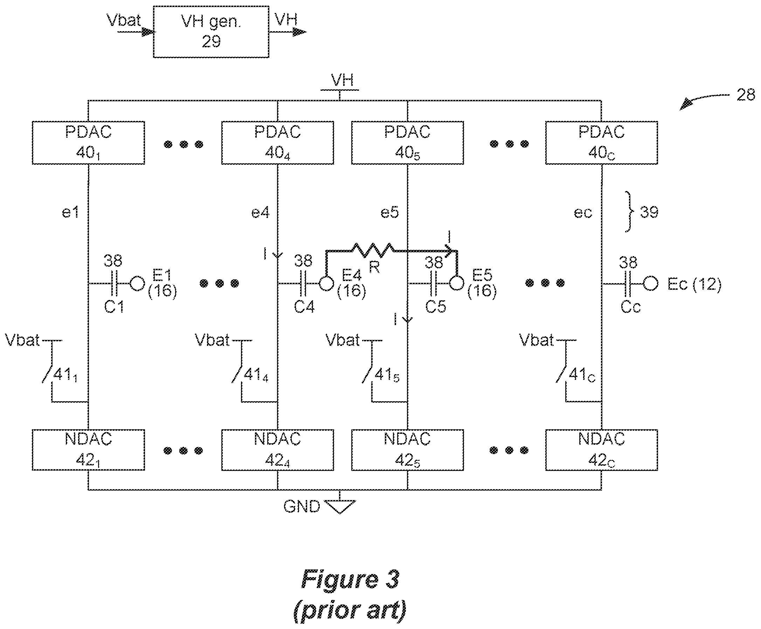

**IPG** 10 as mentioned includes stimulation circuitry 28 to form prescribed stimulation at a patient's tissue. Figure 3 shows an example of stimulation circuitry 28, which includes one or more current source circuits 40ᵢ and one or more current sink circuits 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node ei 39. Each electrode node ei 39 is connected to an electrode Ei 16 via a DC-blocking capacitor Ci 38, for the reasons explained below. The stimulation circuitry 28 in this example also supports selection of the conductive case 12 as an electrode (Ec 12), which case electrode is typically selected for monopolar stimulation. PDACs 40ᵢ and NDACs 42ᵢ can also comprise voltage sources.

Proper control of the PDACs 40ᵢ and NDACs 42ᵢ allows any of the electrodes 16 to act as anodes or cathodes to create a current through a patient's tissue, R, hopefully with good therapeutic effect. In the example shown (Fig. 2A), and during the first phase 30a in which electrodes E4 and E5 are selected as an anode and cathode respectively, PDAC 40₄ and NDAC 42₅ are activated and digitally programmed to produce the desired current, I, with the correct timing (e.g., in accordance with the prescribed frequency F and pulse width PWa). During the second phase 30b (PWb), PDAC 40₅ and NDAC 42₄ would be activated to reverse the polarity of the current. More than one anode electrode and more than one cathode electrode may be selected at one time, and thus current can flow through the tissue R between two or more of the electrodes 16.

Power for the stimulation circuitry 28 is provided by a compliance voltage VH. As described in further detail in U.S. Patent Application Publication 2013/0289665, the compliance voltage VH can be produced by a compliance voltage generator 29, which can comprise a circuit used to boost the battery 14's voltage (Vbat) to a voltage VH sufficient to drive the prescribed current I through the tissue R. The compliance voltage generator 29 may comprise an inductor-based boost converter as described in the '665 Publication, or can comprise a capacitor-based charge pump. Because the resistance of the tissue is variable, VH may also be variable, and can be as high as 18 Volts in one example.

Other stimulation circuitries 28 can also be used in the IPG 10. In an example not shown, a switching matrix can intervene between the one or more PDACs 40ᵢ and the electrode nodes ei 39, and between the one or more NDACs 42ᵢ and the electrode nodes. Switching matrices allow one or more of the PDACs or one or more of the NDACs to be connected to one or more anode or cathode electrode nodes at a given time. Various examples of stimulation circuitries can be found in USPs 6,181,969, 8,606,362, 8,620,436, and U.S. Patent Application Publications 2018/0071520 and 2019/0083796. Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, and 2012/0095519. As explained in these references, ASIC(s) may also contain other circuitry useful in the IPG 10, such as telemetry circuitry (for interfacing off chip with telemetry antennas 27a and/or 27b), the compliance voltage generator 29, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Although not shown, circuitry in the IPG 10 including the stimulation circuitry 28 can also be included in an External Trial Stimulator (ETS) device which is used to mimic operation of the IPG during a trial period and prior to the IPG 10's implantation. An ETS device is typically used after the electrode array 17 has been implanted in the patient. The proximal ends of the leads in the electrode array 17 pass through an incision in the patient and are connected to the externally-worn ETS, thus allowing the ETS to provide stimulation to the patient during the trial period. Further details conceming an ETS device are described in USP 9,259,574 and U.S. Patent Application Publication 2019/0175915.

Referring again to Figure 2A, the stimulation pulses as shown are biphasic, with each pulse at each electrode comprising a first phase 30a followed thereafter by a second phase 30b of opposite polarity. Biphasic pulses are useful to actively recover any charge that might be stored on capacitive elements in the electrode current paths, such as the DC-blocking capacitors 38, the electrode/tissue interface, or within the tissue itself. To recover all charge by the end of the second pulse phase 30b of each pulse (Vc4 = Vc5 = 0V), the first and second phases 30a and 30b are preferably charged balanced at each electrode, with the phases comprising an equal amount of charge but of the opposite polarity. In the example shown, such charge balancing is achieved by using the same pulse width (PWa = PWb) and the same amplitude (|+I| = |-I|) for each of the pulse phases 30a and 30b. However, the pulse phases 30a and 30b may also be charged balance if the product of the amplitude and pulse widths of the two phases 30a and 30b are equal, as is known.

Figure 3 shows that stimulation circuitry 28 can include passive recovery switches 41ᵢ, which are described further in U.S. Patent Application Publications 2018/0071527 and 2018/0140831. Passive recovery switches 41ᵢ may be attached to each of the electrode nodes 39, and are used to passively recover any charge remaining on the DC-blocking capacitors Ci 38 after issuance of the second pulse phase 30b-i.e., to recover charge without actively driving a current using the DAC circuitry. Passive charge recovery can be prudent, because non-idealities in the stimulation circuitry 28 may lead to pulse phases 30a and 30b that are not perfectly charge balanced. Passive charge recovery typically occurs during at least a portion 30c (Fig. 2A) of the quiet periods between the pulses by closing passive recovery switches 41ᵢ. As shown in Figure 3, the other end of the switches 41ᵢ not coupled to the electrode nodes 39 are connected to a common reference voltage, which in this example comprises the voltage of the battery 14, Vbat, although another reference voltage could be used. As explained in the above-cited references, passive charge recovery tends to equilibrate the charge on the DC-blocking capacitors 38 and other capacitive elements by placing the capacitors in parallel between the reference voltage (Vbat) and the patient's tissue. Note that passive charge recovery is illustrated as small exponentially-decaying curves during 30c in Figure 2A, which may be positive or negative depending on whether pulse phase 30a or 30b has a predominance of charge at a given electrode.

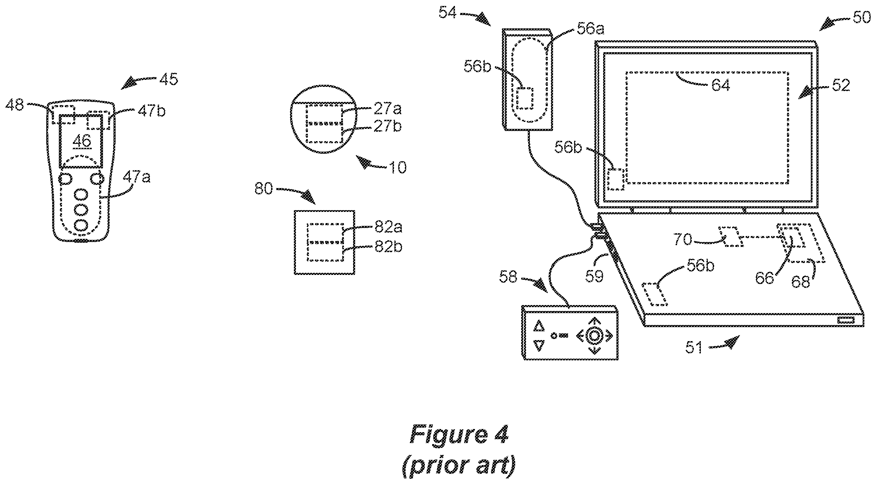

Figure 4 shows various external devices that can wirelessly communicate data with the IPG 10 and/or the ETS 80, including a patient, hand-held external controller 45, and a clinician programmer 50. Both of devices 45 and 50 can be used to wirelessly send a stimulation program to the IPG 10 or ETS 80-that is, to program their stimulation circuitries 28 and 44 to produce pulses with a desired shape and timing described earlier. Both devices 45 and 50 may also be used to adjust one or more stimulation parameters of a stimulation program that the IPG 10 or ETS 80 is currently executing. Devices 45 and 50 may also receive information from the IPG 10 or ETS 80, such as various status information, etc.

External controller 45 can be as described in U.S. Patent Application Publication 2015/0080982 for example, and may comprise either a dedicated controller configured to work with the IPG 10. External controller 45 may also comprise a general purpose mobile electronics device such as a mobile phone which has been programmed with a Medical Device Application (MDA) allowing it to work as a wireless controller for the IPG 10 or ETS 80, as described in U.S. Patent Application Publication 2015/0231402. External controller 45 includes a user interface, including means for entering commands (e.g., buttons or icons) and a display 46. The external controller 45's user interface enables a patient to adjust stimulation parameters, although it may have limited functionality when compared to the more-powerful clinician programmer 50, described shortly.

The external controller 45 can have one or more antennas capable of communicating with the IPG 10 and ETS 80. For example, the external controller 45 can have a near-field magnetic-induction coil antenna 47a capable of wirelessly communicating with the coil antenna 27a or 42a in the IPG 10 or ETS 80. The external controller 45 can also have a far-field RF antenna 47b capable of wirelessly communicating with the RF antenna 27b or 42b in the IPG 10 or ETS 80.

The external controller 45 can also have control circuitry 48 such as a microprocessor, microcomputer, an FPGA, other digital logic structures, etc., which is capable of executing instructions in an electronic device. Control circuitry 48 can for example receive patient adjustments to stimulation parameters, and create a stimulation program to be wirelessly transmitted to the IPG 10 or ETS 80.

Clinician programmer 50 is described further in U.S. Patent Application Publication 2015/0360038. and is only briefly explained here. The clinician programmer 50 can comprise a computing device 51, such as a desktop, laptop, or notebook computer, a tablet, a mobile smart phone, a Personal Data Assistant (PDA)-type mobile computing device, etc. In Figure 4, computing device 51 is shown as a laptop computer that includes typical computer user interface means such as a screen 52, a mouse, a keyboard, speakers, a stylus, a printer, etc., not all of which are shown for convenience. Also shown in Figure 4 are accessory devices for the clinician programmer 50 that are usually specific to its operation as a stimulation controller, such as a communication "wand" 54, and a joystick 58, which are coupleable to suitable ports on the computing device 51, such as USB ports 59 for example.

The antenna used in the clinician programmer 50 to communicate with the IPG 10 or ETS 80 can depend on the type of antennas included in those devices. If the patient's IPG 10 or ETS 80 includes a coil antenna 27a or 82a, wand 54 can likewise include a coil antenna 56a to establish near-filed magnetic-induction communications at small distances. In this instance, the wand 54 may be affixed in close proximity to the patient, such as by placing the wand 54 in a belt or holster wearable by the patient and proximate to the patient's IPG 10 or ETS 80. If the IPG 10 or ETS 80 includes an RF antenna 27b or 82b, the wand 54, the computing device 51, or both, can likewise include an RF antenna 56b to establish communication with the IPG 10 or ETS 80 at larger distances. (Wand 54 may not be necessary in this circumstance). The clinician programmer 50 can also establish communication with other devices and networks, such as the Internet, either wirelessly or via a wired link provided at an Ethernet or network port.

To program stimulation programs or parameters for the IPG 10 or ETS 80, the clinician interfaces with a clinician programmer graphical user interface (GUI) 64 provided on the display 52 of the computing device 51. As one skilled in the art understands, the GUI 64 can be rendered by execution of clinician programmer software 66 on the computing device 51, which software may be stored in the device's non-volatile memory 68. One skilled in the art will additionally recognize that execution of the clinician programmer software 66 in the computing device 51 can be facilitated by controller circuitry 70 such as a microprocessor, microcomputer, an FPGA, other digital logic structures, etc., which is capable of executing programs in a computing device. In one example, controller circuitry 70 can include any of the i5 Core Processors, manufactured by Intel Corp. Such controller circuitry 70, in addition to executing the clinician programmer software 66 and rendering the GUI 64, can also enable communications via antennas 56a or 56b to communicate stimulation parameters chosen through the GUI 64 to the patient's IPG 10.

While GUI 64 is shown as operating in the clinician programmer 50, the user interface of the external controller 45 may provide similar functionality as the external controller 45 may have similar controller circuitry, software, etc.

### SUMMARY

Disclosed herein, but merely as an example and not claimed, is a method of providing electrical stimulation to a patient's neural tissue using an implantable pulse generator (IPG) implanted in the patient and connected to a plurality of electrodes implanted in the patient, the method comprising: using stimulation circuitry of the IPG to cause a first one or more of the plurality of electrodes to provide electrical stimulation to the patient's neural tissue, using sensing circuitry of the IPG to cause a second one or more of the plurality of electrodes to record neural signals in the patient's neural tissue, using control circuitry of the IPG to: extract one or more features of the recorded neural signals, and use the extracted one or more features as reference control variables in a feedback control algorithm to adjust the electrical stimulation based on one or more control algorithm parameters, receiving at the IPG an indication of a state of the patient, using the control circuitry of the IPG to adjust one or more of the control algorithm parameters based on the indication of the state of the patient. According to some embodiments, the one or more features comprise a peak height, a frequency, a peak area, and/or a conduction velocity. According to some embodiments, the state of the patient comprises the patient's posture. According to some embodiments, the state of the patient comprises the patient's sleep state. According to some embodiments, the state of the patient comprises the patient's medication state. According to some embodiments, the indication of the patient state is determined based on a patient survey. According to some embodiments, the indication of the patient state is provided by an accelerometer. According to some embodiments, the accelerometer is configured within the IPG. According to some embodiments, adjusting one or more of the control algorithm parameters comprises adjusting a gain of the feedback control algorithm. According to some embodiments, adjusting one or more of the control algorithm parameters comprises using a different extracted feature as the reference control variable in the feedback control algorithm. According to some embodiments, adjusting one or more of the control algorithm parameters comprises adjusting a setpoint and/or a threshold of the feedback control algorithm. According to some embodiments, adjusting one or more of the control algorithm parameters comprises adjusting a frequency at which the feedback control algorithm determines whether to adjustment to the stimulation. According to some embodiments, adjusting one or more of the control algorithm parameters comprises disabling the feedback control algorithm. According to some embodiments, the indication of the state of the patient indicates the patient's sleep state and wherein using the control circuitry of the IPG to adjust one or more of the control algorithm parameters based on the indication of the state of the patient comprises deactivating the feedback control algorithm if the patient is asleep.

Also disclosed herein is a system for providing electrical stimulation to a patient's neural tissue, the system comprising: an implantable pulse generator (IPG) configured to be implanted in the patient and connected to a plurality of electrodes implanted in the patient, the IPG comprising: stimulation circuitry configured to cause a first one or more of the plurality of electrodes to provide electrical stimulation to the patient's neural tissue, sensing circuitry configured to cause a second one or more of the plurality of electrodes to record neural signals in the patient's neural tissue, control circuitry configured to: extract one or more features of the recorded neural signals, use the extracted one or more features as reference control variables in a feedback control algorithm to adjust the electrical stimulation based on one or more control algorithm parameters, receiving an indication of a state of the patient, adjust one or more of the control algorithm parameters based on the indication of the state of the patient. According to some embodiments, the one or more features comprise a peak height, a frequency, a peak area, and/or a conduction velocity. According to some embodiments, the state of the patient comprises the patient's posture. According to some embodiments, the state of the patient comprises the patient's sleep state. According to some embodiments, the state of the patient comprises the patient's medication state. According to some embodiments, the indication of the patient state is determined based on a patient survey. According to some embodiments, the indication of the patient state is provided by an accelerometer. According to some embodiments, the accelerometer is configured within the IPG. According to some embodiments, adjusting one or more of the control algorithm parameters comprises adjusting a gain of the feedback control algorithm. According to some embodiments, adjusting one or more of the control algorithm parameters comprises using a different extracted feature as the reference control variable in the feedback control algorithm. According to some embodiments, adjusting one or more of the control algorithm parameters comprises adjusting a setpoint and/or a threshold of the feedback control algorithm. According to some embodiments, adjusting one or more of the control algorithm parameters comprises adjusting a frequency at which the feedback control algorithm determines whether to adjustment to the stimulation. According to some embodiments, adjusting one or more of the control algorithm parameters comprises disabling the feedback control algorithm. According to some embodiments, the indication of the state of the patient indicates the patient's sleep state and wherein using the control circuitry of the IPG to adjust one or more of the control algorithm parameters based on the indication of the state of the patient comprises deactivating the feedback control algorithm if the patient is asleep.

Also disclosed herein, but merely as an example and not claimed, is a method of providing electrical stimulation to a patient's neural tissue using an implantable pulse generator (IPG) implanted in the patient and connected to a plurality of electrodes implanted in the patient, the method comprising: using stimulation circuitry of the IPG to cause a first one or more of the plurality of electrodes to provide electrical stimulation to the patient's neural tissue, using sensing circuitry of the IPG to cause a second one or more of the plurality of electrodes to record neural signals in the patient's neural tissue, using control circuitry of the IPG to: extract one or more features of the recorded neural signals, use the extracted one or more features as reference control variables in a feedback control algorithm to adjust the electrical stimulation based on one or more control algorithm parameters, determine at least one optimization metric indicative of the control algorithm's performance, and using the optimization metric to optimize the performance of the feedback control algorithm. According to some embodiments, the at least one optimization metric comprises a frequency at which the feedback control algorithm adjusts the electrical stimulation. According to some embodiments, the optimization metric to optimize the performance of the feedback control algorithm comprises adjusting one or more of the control algorithm parameters based on the optimization metric. According to some embodiments, adjusting one or more of the control algorithm parameters comprises adjusting a gain of the feedback control algorithm. According to some embodiments, adjusting one or more of the control algorithm parameters comprises using a different extracted feature as the reference control variable in the feedback control algorithm. According to some embodiments, adjusting one or more of the control algorithm parameters comprises adjusting a setpoint and/or a threshold of the feedback control algorithm. According to some embodiments, adjusting one or more of the control algorithm parameters comprises adjusting a frequency at which the feedback control algorithm determines whether to adjustment to the stimulation. According to some embodiments, adjusting one or more of the control algorithm parameters comprises disabling the feedback control algorithm. According to some embodiments, the one or more features comprise a peak height, a frequency, a peak area, and/or a conduction velocity.

Also disclosed herein is a system for providing electrical stimulation to a patient's neural tissue, the system comprising: an implantable pulse generator (IPG) configured to be implanted in the patient and connected to a plurality of electrodes implanted in the patient, the IPG comprising: stimulation circuitry configured to cause a first one or more of the plurality of electrodes to provide electrical stimulation to the patient's neural tissue, sensing circuitry configured to cause a second one or more of the plurality of electrodes to record neural signals in the patient's neural tissue, control circuitry configured to: extract one or more features of the recorded neural signals, use the extracted one or more features as reference control variables in a feedback control algorithm to adjust the electrical stimulation based on one or more control algorithm parameters, determine at least one optimization metric indicative of the control algorithm's performance, and use the optimization metric to optimize the performance of the feedback control algorithm. According to some embodiments, the at least one optimization metric comprises a frequency at which the feedback control algorithm adjusts the electrical stimulation. According to some embodiments, using the optimization metric to optimize the performance of the feedback control algorithm comprises adjusting one or more of the control algorithm parameters based on the optimization metric. According to some embodiments, adjusting one or more of the control algorithm parameters comprises adjusting a gain of the feedback control algorithm. According to some embodiments, adjusting one or more of the control algorithm parameters comprises using a different extracted feature as the reference control variable in the feedback control algorithm. According to some embodiments, adjusting one or more of the control algorithm parameters comprises adjusting a setpoint and/or a threshold of the feedback control algorithm. According to some embodiments, adjusting one or more of the control algorithm parameters comprises adjusting a frequency at which the feedback control algorithm determines whether to adjustment to the stimulation. According to some embodiments, adjusting one or more of the control algorithm parameters comprises disabling the feedback control algorithm. According to some embodiments, the one or more features comprise a peak height, a frequency, a peak area, and/or a conduction velocity.

### BRIEF DESCRIPTION OF THE DRAWINGS

Figure 1 shows an Implantable Pulse Generator (IPG), in accordance with the prior art.
Figures 2A and 2B show an example of stimulation pulses producible by the IPG, in accordance with the prior art.
Figure 3 shows stimulation circuitry useable in the IPG, in accordance with the prior art.
Figure 4 shows external devices able to communicate with the IPG, in accordance with the prior art.
Figure 5 shows an improved IPG having stimulation capability and the ability to sense an ElectroSpinoGram (ESG) signal which may include Evoked Compound Action Potentials (ECAPs) caused by the simulation.
Figure 6 shows an example of evoked resonant neural activity (ERNA).
Figure 7 shows an embodiment closed loop feedback control algorithm.
Figure 8 shows an embodiment for adaptively adjusting a closed loop feedback control algorithm.
Figure 9A shows a system wherein a closed loop feedback control algorithm is adjusted based on a diagnostic data log and Figure 9B shows an embodiment of a diagnostic data log.
Figure 10 shows a schematic of an embodiment of an IPG.
Figure 11 shows adjustment of stimulation current by two control algorithms.

### DETAILED DESCRIPTION

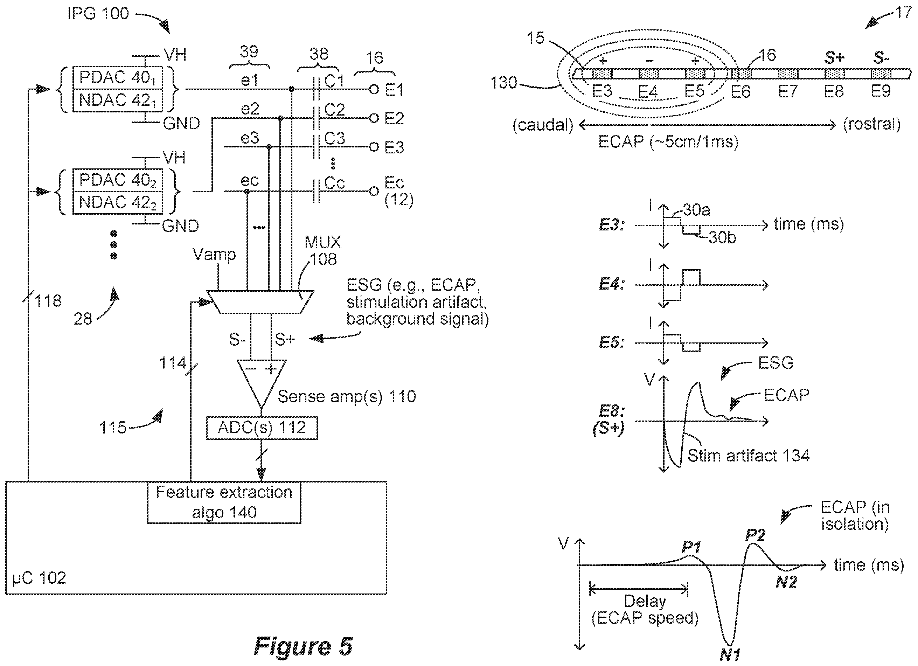

An increasingly interesting development in pulse generator systems, and in Spinal Cord Stimulator (SCS) pulse generator systems specifically, is the addition of sensing capability to complement the stimulation that such systems provide. Figure 5 shows an IPG 100 that includes stimulation and sensing functionality. An ETS as described earlier could also include stimulation and sensing capabilities, and the circuitry shown in Figure 5.

For example, it can be beneficial to sense a neural response in neural tissue that has received stimulation from the IPG 100. One such neural response is an Evoked Compound Action Potential (ECAP). An ECAP comprises a cumulative response provided by neural fibers that are recruited by the stimulation, and essentially comprises the sum of the action potentials of recruited neural elements (ganglia or fibers) when they "fire."

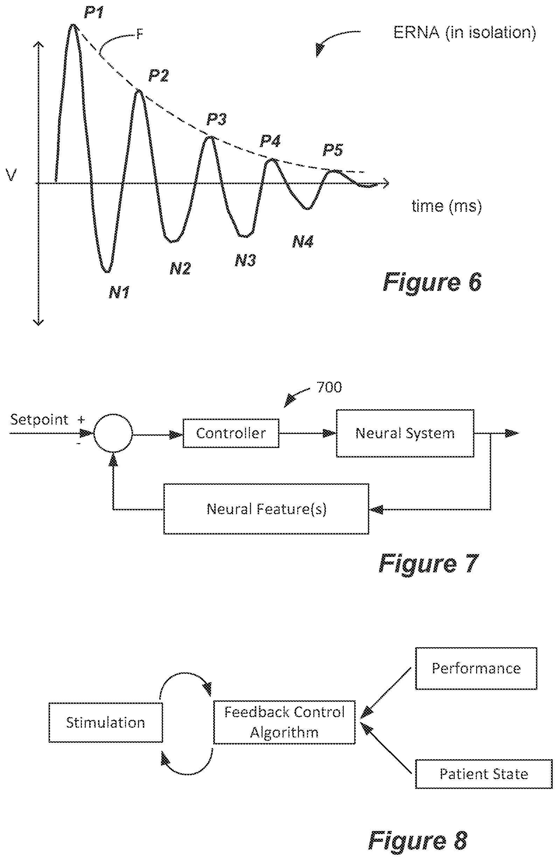

ECAPs are typically associated with stimulation of the spinal column, such as in SCS. It has been observed that stimulation in certain positions in the brain can also evoke neural responses. One example of such neural responses are are resonant neural responses, referred to herein as evoked resonant neural responses (ERNAs). See, e.g., Sinclair, et al., "Subthalamic Nucleus Deep Brain Stimulation Evokes Resonant Neural Activity," Ann. Neurol. 83(5), 1027-31, 2018. The ERNA responses typically have an oscillation frequency of about 200 to about 500 Hz. Stimulation of the STN, and particularly of the dorsal subregion of the STN, has been observed to evoke strong ERNA responses, whereas stimulation of the posterior subthalamic area (PSA) does not evoke such responses. Thus, ERNA may provide a biomarker for electrode location, which can indicate acceptable or optimal lead placement and/or stimulation field placement for achieving the desired therapeutic response. An example of an ERNA in isolation is illustrated in Figure 6. The illustrated ERNA comprises a number of positive peaks Pₙ and negative peaks Nₙ, which may have characteristic amplitudes, separations, or latencies. The ERNA signal may decay according to a characteristic decay function F. Such characteristics of the ERNA response may provide indications of the brain activity associated with the neural response. Other examples of electrical activity/neural responses that may be recorded include motor evoked potentials (MEPs) spontaneous neural activity (local field potentials) as well as other evoked potentials, such as cortical evoked potentials, compound muscle action potentials (CMAPs).

For purposes of this discussion, we will focus on ECAPs as an example of a neural response, though any of the neural responses/electrical activity mentioned above may be used in the context of this disclosure. An ECAP is shown in isolation in Figure 5, and comprises a number of peaks that are conventionally labeled with P for positive peaks and N for negative peaks, with P1 comprising a first positive peak. N1 a first negative peak. P2 a second positive peak, N2 a second negative peak, and so on. Note that not all ECAPs will have the exact shape and number of peaks as illustrated in Figure 5, because an ECAP's shape is a function of the number and types of neural elements that are recruited and that are involved in its conduction. An ECAP is generally a small signal, and may have a peak-to-peak amplitude on the order of hundreds of microvolts or more.

Figure 5 also shows an electrode array 17 comprising (in this example) a single percutaneous lead 15, and shows use of electrodes E3, E4 and E5 to produce pulses in a tripolar mode of stimulation, with (during the first phase 30a) E3 and E5 comprising anodes and E4 a cathode. Other electrode arrangements (e.g., bipoles, etc.) could be used as well. Such stimulation produces an electric field 130 in a volume of the patient's tissue centered around the selected electrodes. Some of the neural fibers within the electric field 130 will be recruited and fire, particularly those proximate to the cathodic electrode E4, forming ECAPs which can travel both rostrally toward the brain and caudally away from the brain. The ECAPs pass through the spinal cord by neural conduction with a speed which is dependent on the neural fibers involved in the conduction. In one example, the ECAP may move at a speed of about 5 cm / 1 ms. U.S. Patent Application Publication 2020/0155019 describes a lead that can be useful in the detection of ECAPs.

ECAPs can be sensed at one or more sensing electrodes which can be selected from the electrodes 16 in the electrode array 17. Sensing preferably occurs differentially, with one electrode (e.g., S+, E8) used for sensing and another (e.g., S-, E9) used as a reference. This could also be flipped, with E8 providing the reference (S-) for sensing at electrode E9 (S+). Although not shown, the case electrode Ec (12) can also be used as a sensing reference electrode S-. Sensing reference S- could also comprise a fixed voltage provided by the IPG 100 (e.g., Vamp, discussed below), such as ground, in which case sensing would be said to be single-ended instead of differential.

The waveform appearing at sensing electrode E8 (S+) is shown in Figure 5, which includes a stimulation artifact 134 as well as a neural response. The stimulation artifact 134 comprises a voltage that is formed in the tissue as a result of the stimulation, i.e., as a result of the electric field 130 that the stimulation creates in the tissue. As described in U.S. Patent Application Publication 2019/0299006, the voltage in the tissue can vary between ground and the compliance voltage VH used to power the DACs, and so the stimulation artifact 134 can be on the order of Volts, and therefore significantly higher than the magnitude of stimulation-induced neural responses. Generally speaking, the waveform sensed at the sensing electrode may be referred to as an ElectroSpinoGram (ESG) signal, which comprises the neural responses, the stimulation artifact 134, and other background signals that may be produced by neural tissue even absent stimulation. Realize that the ESG signal as shown at the sensing electrode S+ in Figure 5 is idealized. The figures in U.S. Patent Application Publication 2022/0323764 show actual recorded ESG traces.

The magnitudes of the stimulation artifact 134 and the neural responses at the sensing electrodes S+ and S- are dependent on many factors, such as the strength of the stimulation, and the distance of sensing electrodes from the stimulation. Some neural responses, such as ECAPs, tend to decrease in magnitude at increasing stimulation-to-sensing distances because they disperse in the tissue. Stimulation artifacts 134 also decrease in magnitude at increasing stimulation-to-sensing distances because the electric field 130 is weaker at further distances. Note that the stimulation artifact 134 is also generally larger during the provision of the pulses, although it may still be present even after the pulse (i.e., the last phase 30b of the pulse) has ceased, due to the capacitive nature of the tissue or the capacitive nature of the driving circuitry (i.e., the DACs). As a result, the electric field 130 may not dissipate immediately upon cessation of the pulse.

It can be useful to sense in the IPG 100 features of either or both of the ECAPs or stimulation artifact 134 contained within the sensed ESG signal, because such features can be used to useful ends. For example, neural response features can be used for feedback, such as closed-loop feedback, to adjust the stimulation the IPG 100 provides. See, e.g., USP 10,406,368; U.S. Patent Application Publications 2019/0099602, 2019/0209844, 2019/0070418, 2020/0147393 and 2022/0347479. It can also be useful to detect features of stimulation artifacts 134 in their own right. For example, U.S. Patent Application Publication 2022/0323764 describes that features of stimulation artifacts can be useful to determining patent posture or activity, which again may then in turn be used to adjust the stimulation that the IPG 100 provides.

Figure 5 shows further details of the circuitry in an IPG 100 that can provide stimulation and sensing an ElectroSpinoGram (ESG) signal. The IPG 100 includes control circuitry 102, which may comprise a microcontroller, such as Part Number MSP430, manufactured by Texas Instruments, Inc., which is described in data sheets at http:// www.ti.com/ microcontrollers/ msp430-ultra-low-power-mcus/ overview.html, which are incorporated herein by reference. Other types of controller circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), such as those described and incorporated earlier. Embodiments of the control circuitry may also be referred to as microcontrollers.

The IPG 100 also includes stimulation circuitry 28 to produce stimulation at the electrodes 16, which may comprise the stimulation circuitry 28 shown earlier (Fig. 3). A bus 118 provides digital control signals from the control circuitry 102 (and possibly from an feature extraction algorithm 140, described below) to one or more PDACs 40ᵢ or NDACs 42ᵢ to produce currents or voltages of prescribed amplitudes (I) for the stimulation pulses, and with the correct timing (PW, F) at selected electrodes. As noted earlier, the DACs can be powered between a compliance voltage VH and ground. As also noted earlier, but not shown in Figure 4, switch matrices could intervene between the PDACs and the electrode nodes 39, and between the NDACs and the electrode nodes 39, to route their outputs to one or more of the electrodes, including the conductive case electrode 12 (Ec). Control signals for switch matrices, if present, may also be carried by bus 118. Notice that the current paths to the electrodes 16 include the DC-blocking capacitors 38 described earlier, which provide safety by preventing the inadvertent supply of DC current to an electrode and to a patient's tissue. Passive recovery switches 41ᵢ (Fig. 3) could also be present, but are not shown in Figure 5 for simplicity.

IPG 100 also includes sensing circuitry 115, and one or more of the electrodes 16 can be used to sense signals the ESG signal. In this regard, each electrode node 39 is further coupleable to a sense amp circuit 110. Under control by bus 114, a multiplexer 108 can select one or more electrodes to operate as sensing electrodes (S+, S-) by coupling the electrode(s) to the sense amps circuit 110 at a given time, as explained further below. Although only one multiplexer 108 and sense amp circuit 110 are shown in Figure 5, there could be more than one. For example, there can be four multiplexer 108/sense amp circuit 110 pairs each operable within one of four timing channels supported by the IPG 100 to provide stimulation. The sensed signals output by the sense amp circuitry are preferably converted to digital signals by one or more Analog-to-Digital converters (ADC(s)) 112, which may sample the output of the sense amp circuit 110 at 50 kHz for example. The ADC(s) 112 may also reside within the control circuitry 102, particularly if the control circuitry 102 has A/D inputs. Multiplexer 108 can also provide a fixed reference voltage, Vamp, to the sense amp circuit 110, as is useful in a single-ended sensing mode (i.e., to set S- to Vamp).

So as not to bypass the safety provided by the DC-blocking capacitors 38, the inputs to the sense amp circuitry 110 are preferably taken from the electrode nodes 39. However, the DC-blocking capacitors 38 will pass AC signal components (while blocking DC components), and thus AC components within the signals being sensed (such as the neural response, stimulation artifact, etc.) will still readily be sensed by the sense amp circuitry 110. In other examples, signals may be sensed directly at the electrodes 16 without passage through intervening capacitors 38.

As noted above, it is preferred to sense an neural response signal differentially, and in this regard, the sense amp circuitry 110 comprises a differential amplifier receiving the sensed signal S+ (e.g., E8) at its non-inverting input and the sensing reference S- (e.g., E9) at its inverting input. As one skilled in the art understands, the differential amplifier will subtract S-from S+ at its output, and so will cancel out any common mode voltage from both inputs. This can be useful for example when sensing neural responses, as it may be useful to subtract the relatively large scale stimulation artifact 134 from the measurement (as much as possible) in this instance. That being said, note that differential sensing will not completely remove the stimulation artifact, because the voltages at the sensing electrodes S+ and S- will not be exactly the same. For one, each will be located at slightly different distances from the stimulation and hence will be at different locations in the electric field 130. Thus, the stimulation artifact 134 can still be sensed even when differential sensing is used. Examples of sense amp circuitry 110, and manner in which such circuitry can be used, can be found in U.S. Patent Application Publications 2019/0299006, 2020/0305744, 2020/0305745 and 2022/0233866.

The digitized sensed signal from the ADC(s) 112-inclusive of any detected neural responses and stimulation artifacts-may be received at a feature extraction algorithm 140 programmed into the IPG's control circuitry 102. The feature extraction algorithm 140 analyzes the digitized sensed signals and may determine one or more neural response features, and/or one or more stimulation artifact features, as described for example in U.S. Patent Application Publication 2022/0323764. Such features may generally indicate the size and shape of the relevant signals, but may also be indicative of other factors (like conduction speed). One skilled in the art will understand that the feature extraction algorithm 140 can comprise instructions that can be stored on non-transitory machine-readable media, such as magnetic, optical, or solid-state memories within the IPG 100 (e.g., stored in association with control circuitry 102).

For example, the feature extraction algorithm 140 can determine one or more neural response features, which may include but are not limited to:
- a height of any peak (e.g., N1);
- a peak-to-peak height between any two peaks (such as from N1 to P2);
- a ratio of peak heights (e.g., N1 / P2);
- a peak width of any peak (e.g., the full-width half-maximum of N1);
- an area or energy under any peak;
- a total area or energy comprising the area or energy under positive peaks with the area or energy under negative peaks subtracted or added;
- a length of any portion of the curve of the neural response (e.g., the length of the curve from P1 to N2);
- any time defining the duration of at least a portion of the neural response (e.g., the time from P1 to N2);
- a time delay from stimulation to issuance of the neural response, which is indicative of the neural conduction speed of the neural response, which can be different in different types of neural tissues;
- a conduction speed (i.e., conduction velocity) of the neural response, which can be determined by sensing the neural response as it moves past different sensing electrodes;
- a rate of variation of any of the previous features, i.e., how such features change over time;
- a power (or energy) determined in a specified frequency band (e.g., delta, alpha, beta, gamma, etc.) determined in a specified time window (for example, a time window that overlaps the neural response, the stimulation artifact, etc.);
- any mathematical combination or function of these variables; and
- frequency domain features, power spectrum, etc., in the context of oscillating neural responses, such as ERNA.

Such neural response features may be approximated by the feature extraction algorithm 140. For example, the area under the curve may comprise a sum of the absolute value of the sensed digital samples over a specified time interval. Similarly, curve length may comprise the sum of the absolute value of the difference of consecutive sensed digital samples over a specified time interval. Neural response features may also be determined within particular time intervals, which intervals may be referenced to the start of simulation, or referenced from within the neural response signal itself (e.g., referenced to peak N1 for example).

The feature extraction algorithm 140 can also determine one or more stimulation artifact features, which may be similar to the neural response features just described, but which may also be different to account for the stimulation artifact 134's different shape. Determined stimulation artifact features may include but are not limited to:
- a height of any peak;
- a peak-to-peak height between any two peaks;
- a ratio of peak heights;
- an area or energy under any peak;
- a total area or energy comprising the area or energy under positive peaks with the area or energy under negative peaks subtracted or added:
- a length of any portion of the curve of the stimulation artifact;
- any time defining the duration of at least a portion of the stimulation artifact;
- a rate of variation of any of the previous features, i.e., how such features change over time;
- a power (or energy) determined in a specified frequency band (e.g., delta, alpha, beta, gamma, etc.) determined in a specified time window (for example, a time window that overlaps the neural response, the stimulation artifact, etc.);
- any mathematical combination or function of these variables.

Again, such stimulation artifact features may be approximated by the feature extraction algorithm 140, and may be determined with respect to particular time intervals, which intervals may be referenced to the start or end of simulation, or referenced from within the stimulation artifact signal itself (e.g., referenced to a particular peak).

Once the feature extraction algorithm 140 determines one or more of these features of the neural response, stimulation artifact and/or other recorded electrical signal, it may then be used to any useful effect in the IPG 100, and specifically may be used to adjust the stimulation that the IPG 100 provides, for example by providing new data to the stimulation circuitry 28 via bus 118. This is explained further in some of the U.S. patent documents cited above. Figure 7 illustrates a simplified closed loop feedback control algorithm 700, whereby a controller seeks to control stimulation of a neural system to maintain one or more neural features with respect to a set point. The control algorithm 700 may be embodied and executed in the control circuitry of the IPG, for example. As mentioned above, the neural system could be the patient's spinal cord (e.g., in the context of SCS), the patient's brain (e.g., in the context of DBS), peripheral nerves, or any other neural system. The set point may be a value, range, or threshold value for one or more neural features that correspond to stimulation providing a therapeutic benefit, lack of side effect, etc. Interrupts may be issued based on a comparison of the one or more neural features with the setpoint. The interrupts may trigger calling or switching between pre-defined stimulation programs. Alternatively, the control scheme may involve controllers such PID controllers, Kalman filters, or the like, which may be configured to adjust one or more stimulation parameters, such as the stimulation current (or voltage) amplitude, pulse width, frequency, or the like.

Closed-loop feedback control is well known in the art and is not discussed here in detail. But a person of skill in the art will understand and appreciate that the performance of the feedback control system depends on many parameters, referred to herein as "control algorithm parameters." Control algorithm parameters are the parameters that define how the control algorithm operates. Examples of control algorithm parameters include the sampling frequency (i.e., how often the neural feature(s) are sampled and compared to the set point), which one or more extracted neural features are used as the reference control variable, set point values, controller gain (i.e., how radically the controller adjusts the stimulation), analog filtering of the sensed neural responses, step size (i.e., how much stimulation can change in one feedback loop), upper/lower limits of stimulation, and the like. Control algorithm parameters such as these should not be confused with stimulation parameters, which are also discussed in this disclosure. As explained above, the stimulation parameters are aspects that characterize the applied stimulation, such as the stimulation current (or voltage) amplitude, pulse width, frequency, applied electric field shape, etc. Aspects of this disclosure relate to using closed loop feedback control (which is executed according to control algorithm parameters) to adjust stimulation by adjusting one or more of the stimulation parameters.

The control algorithm may perform better or worse in some scenarios depending on the control algorithm parameters. For example, in the context of a SCS modality, one set of control algorithm parameters may work best when the patient is in one posture, but another set of control algorithm parameters may work better when the patient is in a different posture. Likewise, the best control algorithm parameters may depend on factors, such as the patient's medication state, tiredness, etc. Stated differently, state changes in the patient (e.g., medication state, posture, etc.) may introduce non-linearities that may not be well-handled by constant control algorithm parameters.

Aspects of this disclosure relate to methods and systems for evaluating the performance of a closed loop feedback control algorithm during the provision of stimulation to a patient and adjusting one or more control algorithm parameters to improve its performance. As illustrated in Figure 8, assume that the stimulator is providing stimulation to a patient under the control of a feedback control algorithm. Aspects of this disclosure involve "controller optimization algorithms" that monitor the feedback control algorithm and adjust the feedback control algorithm to optimize its performance. The controller optimization algorithms may learn how the closed loop feedback controller operates in certain conditions. For example, the feedback control algorithm may be evaluated based on how well it maintains stimulation that is effective at managing the patient's symptoms. Also, the feedback control algorithm may be evaluated based on how often, and how greatly, the feedback control algorithm has to adjust the stimulation. Generally, if the feedback control algorithm is well tuned, the adjustments to the stimulation should be less frequent. However, it may take more frequent and more radical adjustments if the feedback control algorithm undershoots or overshoots the target stimulation. The controller optimization algorithm may be configured to adjust/modify the operation of the feedback controller to provide better control of stimulation. Also, according to some embodiments, the feedback control algorithm may be adjusted based on parameters, such as the patient's state. As mentioned above, some control parameters may work better for some postures, but not for others. Also, the patient's activity level, medication state, etc., may impact which reference neural feature parameters are most suitable.

The disclosed systems and methods may also be implemented when/if the IPG needs to exit a "fallback mode" because of a problem with sensing and/or feedback. As a person of skill in the art will understand, a fallback mode (aka, fail-safe mode) may be a mode of operation (or state) into which the closed loop control system transitions when the closed loop system stops operating due to the detection of a fault. It should be noted that dynamic adjustment of the control algorithm means adjusting the way the algorithm performs its task, not simply adjusting the variable that the algorithm controls. In other words, the dynamic adjustments described herein do not simply adjust the stimulation, but they adjust how the algorithm determines if stimulation needs to be adjusted and/or how the algorithm goes about adjusting the stimulation. The disclosed methods and systems may be implemented in the control circuitry/firmware of the IPG, for example.

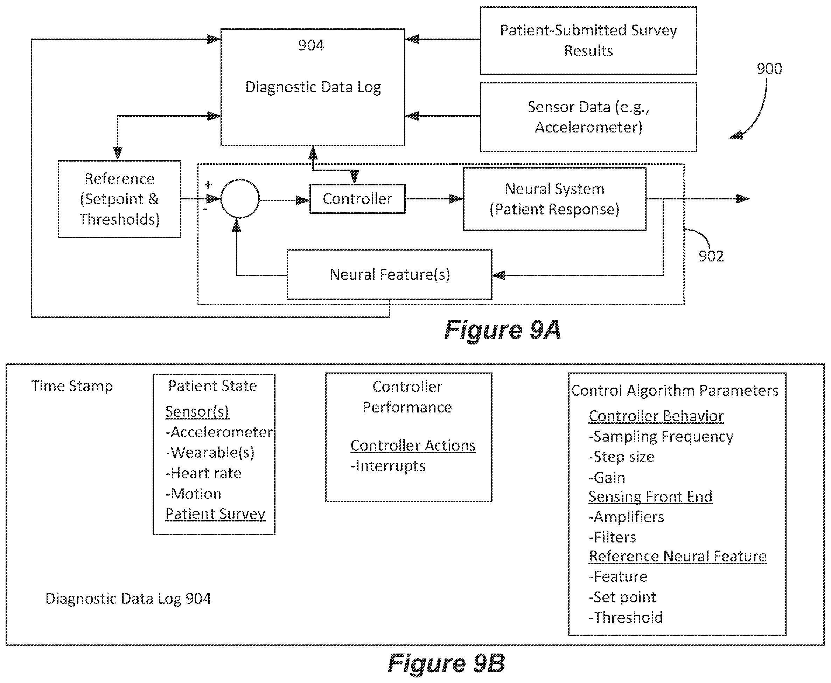

Figures 9A and 9B illustrate an embodiment 900 of a system and and exemplary, not claimed, method for dynamically adjusting parameters of a closed loop feedback control algorithm. The embodiment 900 comprises a closed loop control algorithm 902, which may be as described above with reference to Figure 7. The system also includes a diagnostic data log 904, which is illustrated in more detail if Figure 9B. The diagnostic data log may be configured to log a variety of data relevant to the operation of the closed loop control algorithm, as described further below. According to some embodiments, the logged data may be stored in memory within the IPG. According to some embodiments, the logged data may be transmitted off the IPG, for example, the patient's remote controller (and/or smartphone app) or to the clinician programmer. According to some embodiments, the logged data may ultimately be transmitted to a remote location (e.g., via an internet connection), such as a processing center. According to some embodiments, the sampling/logging of data may be prompted based on a detection of a fault. According to some embodiments, data sampling/logging may be configured to occur periodically, for example, at set intervals. According to some embodiments, data sampling/logging may be prompted based on submitted patient surveys, as explained further below.

Generally, the purpose of the diagnostic data log 904 is to track the logged information about how the control algorithm is performing. The control algorithm's performance may be correlated as a function of the control algorithm parameters. The control algorithm's performance may also be correlated to information indicating the state of the patient. Figure 9B illustrates some examples of the information that may be logged in the diagnostic data log 904. The diagnostic data log may log information from the control and operational circuitry of the IPG, such as time stamps from the IPG's real time clock. According to some embodiments, the time stamps may be correlated with the other data logged in the diagnostic data log.

The diagnostic data log also logs information indicative aspects of the patient's state. According to some embodiments, the patient state information may comprise data from one or more sensors. An example of such a sensor is an accelerometer. The accelerometer may be comprised within the IPG. For example, the IPG may comprise an accelerometer capable of determining the three-dimensional orientation of the IPG (and thus of the patient). Alternatively, the accelerometer may be external, for example, a wearable device, such as a watch, bracelet, etc. The accelerometer data may be indicative of the patient's posture, for example, whether they are standing, sitting, lying down, etc. Another example of a sensor is a motion sensor or inertial measurement unit (IMU). The motion sensor may be wearable or contained within the IPG. According to some embodiments, the motion sensor may be used to detect patient behavior, such as tremor, which is relevant to DBS treatment of movement disorders. Other examples of sensors include heart rate monitors and/or other wearable devices.

According to some embodiments, the diagnostic data log may use information from patient rankings and/or survey results to indicate patient state. For example, the patient may provide information via their external controller and/or smartphone app. The patient may rank the effectiveness of their therapy, for example, by assigning one to five stars or some other indication of satisfaction. According to some embodiments, the patient may provide answers to more detailed questions, for example, by indicating particular times of day or particular activities during which they notice a decline in therapeutic efficacy. In embodiments wherein the diagnostic data log is configured with the IPG, the indication of patient survey data may be transmitted to the IPG via a data link, such as BLE connection, for example.

According to some embodiments, patient state may be inferred based on time stamp information. For example, pattern recognition algorithms may be used to correlate the patient's activity level (as measured using accelerometers, motion sensors, etc.) with the time of day. Accordingly, the diagnostic data log may learn to associate various times of day with typical patient states.

The diagnostic data log 904 also monitors the performance of the closed loop feedback control algorithm. For example, the diagnostic data log 904 may log the number of interrupts the feedback control algorithm generates indicating that the reference control variable is outside of a set boundary or threshold value. Interrupts are indicative of number/frequency of corrections. Interrupts may indicate that the control algorithm simply needs to be active during this time. However, a larger number of interrupts may indicate that the feedback control algorithm is having difficulty maintaining the reference control variable (i.e., the reference neural feature) within the set range. Difficulty maintaining the setpoint (reference control variable) would be indicated by a consistent or average deviation of the measured feature level compared with the reference. According to some embodiments, this may be measured by mean squared error level or by % of time spent outside thresholds.

The diagnostic data log 904 also monitors the feedback control algorithm parameters and correlates the feedback control algorithm performance to those parameters. For example, the diagnostic data log may log information indicating the controller's sampling frequency (how often measurements are taken), adjustment step size, and/or gain. The diagnostic data log may also log information indicating operational parameters relating to the operation of the IPG's analog and/or digital sensing circuitry, such as filtering that is applied to the sensed/recorded signals, amplifier gains, averaging, sampling frequency, etc. The diagnostic data log may also log information about which reference neural feature(s) (e.g., peak amplitude, curve length, etc.) are extracted and monitored as the feedback variable.

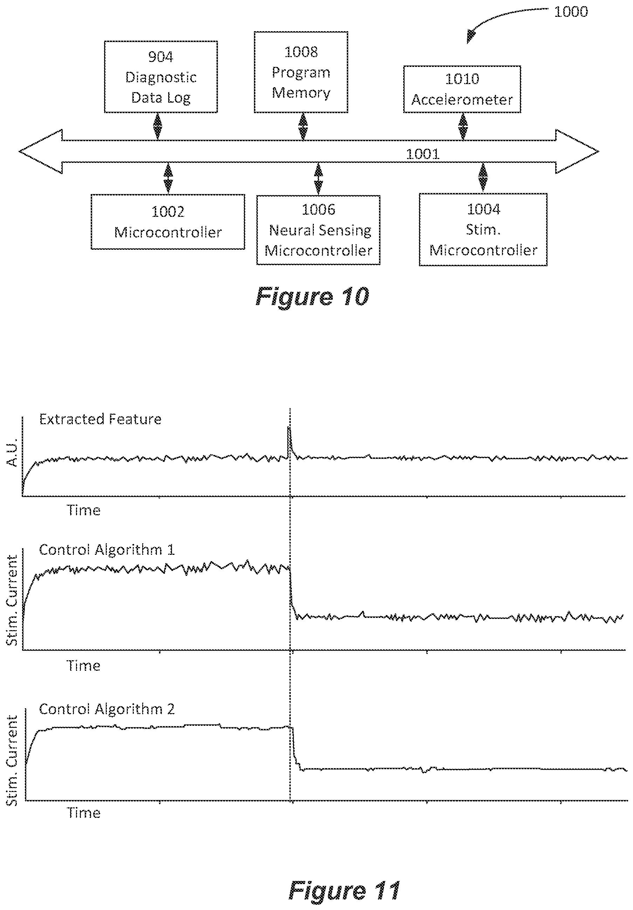

Figure 10 shows a conceptual diagram of a system 1000 that uses a diagnostic data log 904 to dynamically adapt closed loop feedback control of a patient's stimulation. The system 1000 may be embodied in an IPG (e.g., 10, Fig. 1), for example. The system includes a data bus 1001, which is configured to allow the component modules to communicate with each other. The system includes a microcontroller 1002, which may be the microcontroller/processor that controls the overall operation of the IPG. The illustrated system 1000 also includes a stimulation microcontroller 1004 that is configured to control aspects of stimulation, such as the delivery of stimulation according to stimulation parameters, as described above. The system also includes a neural sensing microcontroller 1006 that is configured to control how the IPG senses potentials within the physiological environment of the electrode leads. For example, the neural sensing microcontroller 1006 may control the timing of sensing, which electrodes are used for sensing, what neural features are extracted from the sensed neural responses, front end amplification, and the like. According to some embodiments, the neural sensing microcontroller and/or the stimulation microcontroller may be a single microcontroller or may be included in the overall microcontroller 1002.

The system 1000 also comprises a program memory 1008, which may be configured to contain programs for the operation of the IPG, include stimulation, sensing, etc. For example, the program memory may store various stimulation programs, sensing programs, and feedback algorithms, etc. According to some embodiments, the methods and algorithms described herein for dynamically adapting the feedback control may be stored within the program memory 1006. The programs and algorithms stored within the program memory may be executed using the microcontrollers 1002, 1006, and/or 1004.

As mentioned above, the system 1000 also includes a diagnostic data log 904, as described with regard to Figures 9A and 9B. The diagnostic data log may be configured within memory of the IPG, for example. The system 1000 may also comprises one or more sensors, such as an accelerometer 1010. Data from the sensors may be provided to the diagnostic data log 904, as described above.

While the system provides stimulation under closed loop control, the system (i.e., one or more of the microcontrollers) may execute algorithms to monitor the diagnostic data log 904 (Fig. 9B) and determine relationships between the various information contained in the log. According to some embodiments, the controller optimization algorithms may monitor the controller performance and execute learning algorithms to relate the controller performance to other information in the diagnostic data log. For example, the algorithms may determine relationships between the controller's performance and the particular control algorithm parameters, such as sampling frequency, step size, gain, filtering, the extracted neural feature(s), set points, etc. The learning algorithms may also determine how the control algorithm performs as a function of various patient state indicators, such as the patient's activity and/or posture (as indicated via accelerometer measurements), heart rate, survey results, etc. The system may adjust the control algorithm parameters to improve the control algorithm's performance.

Figure 11 illustrates an example of optimizing the closed loop feedback control algorithm. Trace 1102 shows values (in arbitrary units (A.U.)) of a reference parameter extracted from a neural response measurement during the provision of stimulation under closed loop feedback control. For example, the reference parameter may be extracted from an ECAP, ERNA, local field potential, etc., and may be a height of any peak (e.g., N1), a peak-to-peak height between any two peaks (such as from N1 to P2), or any other reference parameter/value described above. The feedback control algorithm monitors the neural response reference parameter and adjusts the stimulation to maintain the parameter with respect to a threshold, within a range, etc. In the illustrated example, the feedback control algorithm adjusts the stimulation current, though it could adjust other aspects of the stimulation. Notice that at a time denoted 1103, the value of the reference parameter changes (in this case, it sharply increases). Traces 1104 and 1106 show how two different feedback control algorithms may adjust the stimulation current in response to the change in the value of the reference parameter. Both feedback control algorithms lower the stimulation current at the time 1103, which results in the extracted feature returning to values similar to those before the time 1103.

Notice that the control algorithm 1 (1104) is noisier that control algorithm 2 (1106). The "noise" in control algorithm 1 indicates that the algorithm is making many more micro-adjustments to the stimulation current. Each of those adjustments require computing and energy resources. Consequently, it may be concluded that control algorithm 2 is the more efficient, or "optimized" of the two feedback control algorithms.

Embodiments of the controller optimization algorithm may be configured to monitor the closed loop feedback controller's behavior and to adjust the feedback control parameters. According to some embodiments, the controller optimization algorithm may monitor the number of adjustments (e.g., interrupts) the control algorithm issues. If that number exceeds a predetermined threshold, then the controller optimization algorithm may adjust one or more of the feedback control parameters.

As a hypothetical example, consider a situation in which SCS is being used to provide electrical stimulation to a patient's spinal cord. Assume that closed loop feedback control is being used and that the closed loop feedback algorithm uses the area under the curve of sensed ECAP signal as a reference control variable to maintain the stimulation. Assume that over time, the adjustments to the stimulation become more frequent, such that the number of adjustments exceed a predetermined threshold. This may occur because the electrode leads have migrated, scar tissue has formed, or due to some other change in the patient's state. In this example, the behavior of the closed loop feedback control algorithm may resemble trace 1104 (Fig. 11). The controller optimization algorithm may detect this behavior and change the way the closed loop feedback control algorithm operates. For example, the controller optimization algorithm may cause the closed loop feedback control algorithm to switch to using curve length as the control reference variable instead of using area under the curve, in an attempt to achieve behavior more like that shown in trace 1106. According to other embodiments, the controller optimization algorithm may adjust the closed loop feedback control algorithm based on indications of the patient's state, for example, posture, medication state, and the like.

According to some embodiments, the controller optimization algorithm may determine that closed loop feedback control is simply not needed under certain conditions and may simply disable closed loop feedback control for a time. For example, closed loop feedback control may not be worth the energy/computing resource drain, for example, if the patient is asleep. For example, according to some embodiments, sensing and/or closed loop control may be disabled when the patient is asleep.

According to some embodiments, the patient's sleep state may be inferred based on accelerometer data. As mentioned above, the accelerometer may be configured within the IPG, or it may be wearable or otherwise attached to the patient. The controller optimization algorithm may be configured to determine periods when there is little or no change in the accelerometer (e.g., the x, y. and/or z axes) and classify those periods as sleep states. According to some embodiments, if there is little or no accelerometer activity for longer than a predetermined threshold value the period may be classified as a sleep state.

As mentioned above, the closed loop feedback control algorithm may be disabled during a sleep state, for example, to conserve battery life. Alternatively, the closed loop feedback control algorithm and/or the controller optimization algorithm may be modified during a sleep state. For example, during a sleep state the feedback control algorithm may remain abled, but may simply make few measurement and/or make fewer adjustments compared to the wake state. This allows the system to be very responsive to sudden changes in the patient state, such as if the patient coughs, etc.

It should be noted that embodiments described herein involve an IPG that comprises various types of circuitry, such as control circuitry, stimulation circuitry, sensing circuitry, and the like. A person of skill in the art will appreciate that the various types of circuitry may be embodied as separate components, or they may be embodied as pieces of unified circuitry. For example, the stimulation circuitry and/or the sensing circuitry may be embodied as aspects of the control circuitry.

## Claims

1. A system for providing electrical stimulation to a patient's neural tissue, the system comprising:
an implantable pulse generator, IPG, (10) configured to be implanted in the patient and connected to a plurality of electrodes (16) implanted in the patient, the IPG (10) comprising:
stimulation circuitry (28) configured to cause a first one or more of the plurality of electrodes (16) to provide electrical stimulation to the patient's neural tissue,
sensing circuitry (115) configured to cause a second one or more of the plurality of electrodes (16) to record neural signals in the patient's neural tissue,
control circuitry (102) configured to:
extract one or more features of the recorded neural signals,
use the extracted one or more features as reference control variables in a feedback control algorithm to adjust the electrical stimulation based on one or more control algorithm parameters,
receive an indication of a state of the patient, and
adjust one or more of the control algorithm parameters based on the indication of the state of the patient.

2. The system of claim 1, wherein the one or more features comprise a peak height, a frequency, a peak area, and/or a conduction velocity.

3. The system of claims 1 or 2, wherein the state of the patient comprises the patient's posture.

4. The system of any of claims 1-3, wherein the state of the patient comprises the patient's sleep state.

5. The system of any of claims 1-4, wherein the state of the patient comprises the patient's medication state.

6. The system of any of claims 1-5, wherein the indication of the patient state is determined based on a patient survey.

7. The system of any of claims 1-6, wherein the indication of the patient state is provided by an accelerometer.

8. The system of claim 7, wherein the accelerometer is configured within the IPG.

9. The system of any of claims 1-8, wherein adjusting one or more of the control algorithm parameters comprises adjusting a gain of the feedback control algorithm.

10. The system of any of claims 1-9, wherein adjusting one or more of the control algorithm parameters comprises using a different extracted feature as the reference control variable in the feedback control algorithm.

11. The system of any of claims 1-10, wherein adjusting one or more of the control algorithm parameters comprises adjusting a setpoint and/or a threshold of the feedback control algorithm.

12. The system of any of claims 1-11, wherein adjusting one or more of the control algorithm parameters comprises adjusting a frequency at which the feedback control algorithm determines whether to adjustment to the stimulation.

13. The system of any of claims 1-12, wherein adjusting one or more of the control algorithm parameters comprises disabling the feedback control algorithm.

14. The system of any of claims 1-13, wherein the indication of the state of the patient indicates the patient's sleep state and wherein using the control circuitry of the IPG to adjust one or more of the control algorithm parameters based on the indication of the state of the patient comprises deactivating the feedback control algorithm if the patient is asleep.

## Patentansprüche

1. System zum elektrischen Stimulieren von Nervengewebe eines Patienten, wobei das System aufweist:
einen implantierbaren Impulsgenerator, IPG, (10), der dafür konfiguriert ist, in den Patienten implantiert zu werden, und mit einer Mehrzahl von im Patienten implantierten Elektroden (16) verbunden ist, wobei der IPG (10) aufweist:
eine Stimulationsschaltung (28), die dafür konfiguriert ist, zu veranlassen, dass eine erste oder mehrere der Mehrzahl von Elektroden (16) eine elektrische Stimulation für das Nervengewebe des Patienten bereitstellen;
eine Erfassungsschaltung (115), die dafür konfiguriert ist, zu veranlassen, dass eine zweite oder mehrere der Mehrzahl von Elektroden (16) Nervensignale im Nervengewebe des Patienten aufzeichnen;
eine Steuerschaltung (102), die dafür konfiguriert ist:
ein oder mehrere Merkmale der aufgezeichneten Nervensignale zu extrahieren, das eine oder die mehreren extrahierten Merkmale als Referenzsteuervariablen in einem Rückkopplungssteuerungsalgorithmus zu verwenden, um die elektrische Stimulation basierend auf einem oder mehreren Steuerungsalgorithmusparametern anzupassen;
eine Anzeige des Zustands des Patienten zu empfangen; und
einen oder mehrere der Steuerungsalgorithmusparameter basierend auf der Anzeige des Zustands des Patienten anzupassen.

2. System nach Anspruch 1, wobei das eine oder die mehreren Merkmale eine Peakhöhe, eine Frequenz, eine Peakfläche und/oder eine Leitungsgeschwindigkeit aufweisen.

3. System nach Anspruch 1 oder 2, wobei der Zustand des Patienten die Körperhaltung des Patienten aufweist.

4. System nach einem der Ansprüche 1 bis 3, wobei der Zustand des Patienten den Schlafzustand des Patienten aufweist.

5. System nach einem der Ansprüche 1 bis 4, wobei der Zustand des Patienten den Medikationsstatus des Patienten aufweist.

6. System nach einem der Ansprüche 1 bis 5, wobei die Anzeige des Zustands des Patienten basierend auf einer Patientenbefragung bestimmt wird.

7. System nach einem der Ansprüche 1 bis 6, wobei die Anzeige des Zustands des Patienten durch einen Beschleunigungsmesser bereitgestellt wird.

8. System nach Anspruch 7, wobei der Beschleunigungsmesser innerhalb des IPG konfiguriert ist.

9. System nach einem der Ansprüche 1 bis 8, wobei das Anpassen eines oder mehrerer der Steuerungsalgorithmusparameter das Anpassen einer Verstärkung des Rückkopplungssteuerungsalgorithmus aufweist.

10. System nach einem der Ansprüche 1 bis 9, wobei das Anpassen eines oder mehrerer der Steuerungsalgorithmusparameter das Verwenden eines anderen extrahierten Merkmals als Referenzsteuervariable im Rückkopplungssteuerungsalgorithmus aufweist.

11. System nach einem der Ansprüche 1 bis 10, wobei das Anpassen eines oder mehrerer der Steuerungsalgorithmusparameter das Anpassen eines Sollwerts und/oder eines Schwellenwerts des Rückkopplungssteuerungsalgorithmus aufweist.

12. System nach einem der Ansprüche 1 bis 11, wobei das Anpassen eines oder mehrerer der Steuerungsalgorithmusparameter das Anpassen einer Frequenz aufweist, mit der der Rückkopplungssteuerungsalgorithmus bestimmt, ob eine Anpassung der Stimulation erfolgen soll.

13. System nach einem der Ansprüche 1 bis 12, wobei das Anpassen eines oder mehrerer der Steuerungsalgorithmusparameter das Deaktivieren des Rückkopplungssteuerungsalgorithmus aufweist.

14. System nach einem der Ansprüche 1 bis 13, wobei die Anzeige des Zustands des Patienten den Schlafzustand des Patienten anzeigt, und wobei die Verwendung der Steuerschaltung des IPG zum Anpassen eines oder mehrerer der Steuerungsalgorithmusparameter basierend auf der Anzeige des Zustands des Patienten das Deaktivieren des Rückkopplungssteuerungsalgorithmus aufweist, wenn der Patient schläft.

## Revendications

1. Système destiné à fournir une stimulation électrique au tissu neuronal d'un patient, le système comprenant:
un générateur d'impulsions implantable, IPG, (10) configuré pour être implanté chez le patient et connecté à une pluralité d'électrodes (16) implantées chez le patient, l'IPG (10) comprenant:
des circuits de stimulation (28) configurés pour amener une première ou plusieurs de la pluralité d'électrodes (16) à fournir une stimulation électrique au tissu neuronal du patient,
des circuits de détection (115) configurés pour amener une deuxième ou plusieurs de la pluralité d'électrodes (16) à enregistrer des signaux neuronaux dans le tissu neuronal du patient,
des circuits de commande (102) configurés pour:
extraire une ou plusieurs caractéristiques des signaux neuronaux enregistrés,
utiliser les une ou plusieurs caractéristiques extraites comme variables de commande de référence dans un algorithme de commande à rétroaction pour ajuster la stimulation électrique sur la base d'un ou plusieurs paramètres de l'algorithme de commande,
recevoir une indication d'un état du patient, et
ajuster un ou plusieurs des paramètres de l'algorithme de commande sur la base de l'indication de l'état du patient.

2. Système selon la revendication 1, dans lequel les une ou plusieurs caractéristiques comprennent une hauteur de pic, une fréquence, une aire de pic et/ou une vitesse de conduction.

3. Système selon la revendication 1 ou 2, dans lequel l'état du patient comprend la posture du patient.

4. Système selon l'une quelconque des revendications 1 à 3, dans lequel l'état du patient comprend l'état de sommeil du patient.

5. Système selon l'une quelconque des revendications 1 à 4, dans lequel l'état du patient comprend l'état de médication du patient.

6. Système selon l'une quelconque des revendications 1 à 5, dans lequel l'indication de l'état du patient est déterminée sur la base d'un questionnaire destiné au patient.

7. Système selon l'une quelconque des revendications 1 à 6, dans lequel l'indication de l'état du patient est fournie par un accéléromètre.

8. Système selon la revendication 7, dans lequel l'accéléromètre est configuré à l'intérieur de l'IPG.

9. Système selon l'une quelconque des revendications 1 à 8, dans lequel l'ajustement d'un ou plusieurs des paramètres de l'algorithme de commande comprend l'ajustement d'un gain de l'algorithme de commande à rétroaction.

10. Système selon l'une quelconque des revendications 1 à 9, dans lequel l'ajustement d'un ou plusieurs des paramètres de l'algorithme de commande comprend l'utilisation d'une caractéristique extraite différente comme variable de commande de référence dans l'algorithme de commande à rétroaction.

11. Système selon l'une quelconque des revendications 1 à 10, dans lequel l'ajustement d'un ou plusieurs des paramètres de l'algorithme de commande comprend l'ajustement d'une valeur de consigne et/ou d'un seuil de l'algorithme de commande à rétroaction.

12. Système selon l'une quelconque des revendications 1 à 11, dans lequel l'ajustement d'un ou plusieurs des paramètres de l'algorithme de commande comprend l'ajustement d'une fréquence à laquelle l'algorithme de commande à rétroaction détermine s'il convient d'ajuster à la stimulation.

13. Système selon l'une quelconque des revendications 1 à 12, dans lequel l'ajustement d'un ou plusieurs des paramètres de l'algorithme de commande comprend la désactivation de l'algorithme de commande à rétroaction.

14. Système selon l'une quelconque des revendications 1 à 13, dans lequel l'indication de l'état du patient indique l'état de sommeil du patient et dans lequel l'utilisation des circuits de commande de l'IPG pour ajuster un ou plusieurs des paramètres de l'algorithme de commande sur la base de l'indication de l'état du patient comprend la désactivation de l'algorithme de commande à rétroaction si le patient dort.
